# EUROPEAN PATENT SPECIFICATION

(11) **EP 4 634 823 B1**
(45) Date of publication and mention of the grant of the patent: **08.07.2026**
(21) Application number: 23825615.0
(22) Date of filing: 11.12.2023
(51) Int. Cl.: G06N 3/02, G06V 10/82, G06V 40/10, G06N 3/044, G06N 3/0464, G06N 3/084, G06N 3/09, G06V 10/46, G06V 10/774, G06N 3/048

(54) **DETERMINING THE BODY POSTURE OF A LAYING INFANT**
BESTIMMUNG DER KÖRPERHALTUNG EINES LIEGENDEN KLEINKINDES
DÉTERMINATION DE LA POSTURE DU CORPS D'UN NOURRISSON EN POSE

(30) Priority: 13.12.2022 EP 22213168
(43) Date of publication of application: 22.10.2025
(73) Proprietor: Koninklijke Philips N.V., 5656 AE Eindhoven (NL)
(72) Inventor: MAITY, Reevu, 5656 AG Eindhoven (NL); PATIL, Ravindra Balasaheb, 5656 AG Eindhoven (NL); KOCKX, Franciscus Nicolaas, 5656 AG Eindhoven (NL); GUTTA, Srinivas, 5656 AG Eindhoven (NL)
(74) Representative: Philips Intellectual Property & Standards
(86) International application number: PCT/EP2023/085008
(87) International publication number: WO 2024/126318

(56) References cited:
- US-A1- 2018 035 082
- LI MIN ET AL: "Three-Dimensional Pose Estimation of Infants Lying Supine Using Data From a Kinect Sensor With Low Training Cost", IEEE SENSORS JOURNAL, IEEE, USA, vol. 21, no. 5, 10 November 2020 (2020-11-10), pages 6904 - 6913, XP011836170, ISSN: 1530-437X, [retrieved on 20210204], DOI: 10.1109/JSEN.2020.3037121

## Description

### FIELD OF THE INVENTION

This invention relates to the field of infant monitoring, and in particular to determining the body posture of a laying infant.

### BACKGROUND OF THE INVENTION

Some of the major issues related to infant healthcare includes immunization, feeding, sleeping and sudden infant death syndrome (SIDS). SIDS is the sudden and unexplained death of an infant and it has been observed that most SIDS deaths are associated with sleep (which is why SIDs is also termed as 'crib death').

Recent research has identified a link between SIDS and infants who sleep on their belly. It is also recommended not to place the infant to sleep on the side since the infant can roll over to the belly position during sleep. One of the primary concerns of an infant sleeping on the belly is the nasal airway might get blocked during sleep resulting in 'rebreathing', i.e., breathing one's own exhaled air. If an infant keeps rebreathing exhaled air for a prolonged period of time, the oxygen level in the body drops with a consequent rise in the carbon dioxide levels. Infants succumbing to SIDS may have a complication with the part of the brain which deals with breathing control and waking up during sleep.

As a consequence, there is a continued need to develop improved infant monitoring concepts, particularly for monitoring the sleep posture of an infant with the aim of reducing the risks of succumbing to SIDS (e.g. by alerting the parents if their infant is laying in a high-risk posture).

US 2018/035082 A1 describes a monitoring system including a sensor, a processing block and an alert generator. The sensor is used to generate images of an infant, and the processing block processes one or more of the images.

Li Min et al., "Three-Dimensional Pose Estimation of Infants Lying Supine Using Data from a Kinect Sensor With Low Training Cost*",* describes that early diagnosis of cerebral palsy in infants has produced promising results using tools like the General Movement Assessment (GMA).

### SUMMARY OF THE INVENTION

The invention is defined by the claims.

According an aspect of the invention, there is provided a method for determining the body posture of a laying infant. The method comprises: processing an input image of the laying infant with an object detection model to detect the infant's head and upper torso; determining a body axis based on the detected head and upper torso, wherein the body axis describes the longitudinal direction of the upper torso of the infant; processing the input image with a first keypoint detection model to detect and identify at least one shoulder of the infant; determining an orientation of the identified shoulder(s) with respect to the body axis; and classifying the infant's body posture into one of a plurality of body posture classifications based on the determined orientation of the identified shoulder(s) relative to the body axis.

Proposed concepts thus aim to provide schemes, solutions, concept, designs, methods and systems pertaining to identifying and monitoring the body posture of a laying infant (e.g. an infant laying on a bed or cot surface). In particular, embodiments aim to provide a method to identify the posture of a laying infant from processing a single input image of the laying infant.

In other words, it is proposed that a single input image of laying infant may be analysed, in order to obtain a prediction of the infant's posture within the image. Such an input image may be obtained by a device that a user may commonly already have access to, such as a (camera-based) infant monitor, webcam, or smartphone.

By only needing a single input image, embodiments may support identification of an infant's laying posture without any specialised hardware. An improved method for detecting and monitoring the body posture of laying infant may therefore be provided by proposed concepts.

For instance, by automatically processing an input image of a laying infant in order to determine the longitudinal direction of the upper torso of the infant (i.e. body axis) and to detect and identify at least one shoulder of the infant, a prediction of the infant's body posture may be determined by analysing the orientation of the detected and identified shoulder(s) relative to the direction of the infant's upper torso. That is, it is proposed that determination of the infant/s laying posture may be made by analysing the orientation of the infant's shoulder(s) relative to the infant's body axis (in a captured image of the laying infant). Such a proposed approach may be simple but robust to the positioning of the image capture device.

The use of orientation of an infant's shoulder(s) relative to the infant's body axis in a captured image (or video) may permit, for instance, simple identification of the infant's laying posture without the use of any special hardware, i.e., with only a conventional video camera, webcam, or smartphone.

Embodiments of the invention may be of particular use in the case of monitoring a sleeping infant for the purpose of alerting a carer if the infant lays with a posture associated with a high risk of SIDS. In this regard, it is noted that infants are typically covered with heavy garments and heavy blankets during sleep. A method capable of detecting the body posture of a laying infant that is robust/agnostic to an infant's coverage by garments/blankets and only requires a single image would therefore be of great use.

Ultimately, an improved method for determining the body posture of a laying infant may be supported by the proposed concept(s).

In some embodiments, processing an input image of the laying infant with an object detection model to detect infant's head and upper torso may comprise: processing the input image of the laying infant with an object detection model to generate a bounding box for each of the infant's head and upper torso respectively; and wherein determining a body axis based on the detected head and upper torso may comprise: determining a body axis based on the generated boundary boxes, such that the body axis passes through the center of mass of each bounding box.

Some embodiments may further comprise determining an orientation of the body axis. Determining the infant's body posture may be further based on the determined orientation of the body axis. That is, an orientation of the body axis with respect to a viewing angle of the image may be determined and taken account of when determining the infant's body posture. In this way, embodiments may cater for whether an infant lies with its head towards the image capture device or away from the capture device (which may result in left and right being interchanged).

Some embodiments may further comprise processing the input image with a second keypoint detection model to detect one or more facial features of the infant. Determining the infant's body posture may then be further based on the detected one or more facial features. For example, determining the infant's body posture may comprise: determining if the detected one or more facial features of the infant comprises eyes or nose of the infant; and classifying the infant's body posture into one of a plurality of body posture classifications responsive to determining the detected one or more facial features of the infant comprises eyes or nose of the infant. Exemplary body posture classifications may, for instance, comprise: back; belly; and side. In this way, an embodiment may be employed to detect three laying/sleeping positions of a laying infant.

Some embodiments may further comprise: processing the input image with the first keypoint detection model to detect and identify at least one hand of the infant. Determining the infant's body posture may then be further based on the determined body axis and the orientation of detected and identified hand(s) relative to the body axis. By identifying and using the position of at least one hand of the infant, such embodiments may further improve accuracy of posture detection.

In an embodiment, processing an input image of the infant with the object detection model may comprise: feeding the input image to a first machine learning model wherein the first machine learning model is trained to predict co-ordinates of bounding boxes representing the spatial location of the infant's head and upper torso in the input image. By way of example, the first machine learning model is trained using a deep learning algorithm configured to receive an array of training inputs and respective known outputs. A training input may comprise a training image of an infant laying or sleeping in a bed and a respective known output may comprise the co-ordinates of the bounding boxes representing the spatial location of the infant's head and upper torso in the training image. In this way, the first machine learning model may be trained to output an estimation of the position of the infant's head and upper torso when provided with an image of the laying infant.

In some embodiments, processing the input image with the first keypoint detection model may comprise: feeding the input image to a second machine learning model, wherein the second machine-learning model is trained to identify and predict the co-ordinates representing the spatial location of the infant's shoulder(s) and hands in the input image. For instance, the second machine learning model may be trained using a deep learning algorithm configured to receive an array of training inputs and respective known outputs, and a training input may comprise a training image of an infant laying or sleeping in a bed and a respective known output may comprise the co-ordinates of the infant's shoulder and hand keypoints in the training image. In this way, the first keypoint detection model may be trained to output an estimation of the position of the infant's shoulder(s) and hands when provided with an image of the laying infant.

Also, in some embodiments, processing the input image with the second keypoint detection model may comprise: feeding the input image to a third machine learning model wherein the third machine learning model is trained to identify and predict the co-ordinates representing the spatial location of one or more facial features of the infant in the input image. By way of example, the third machine learning models may be trained using a deep learning algorithm configured to receive an array of training inputs and respective known outputs. A training input may comprise a training image of an infant lying down or sleeping in a bed and a respective known output may comprise the co-ordinates of the infant's facial feature keypoints such as eyes, nose, and ears in the training image. In this way, the second keypoint detection model may be trained to output an estimation of the position of the infant's facial features when provided with an image of the laying infant.

In an embodiment, at least one of the first, second and third machine learning models may comprise a pretrained model further trained and fine-tuned on manually annotated images of an infant lying down or sleeping in a bed. This may allow the machine learning model(s) to become especially proficient at predicting the position of body parts/features of an infant.

Thus, machine learning, or artificial intelligence, may be leveraged to support detection of a laying infant's body posture. This may, in turn, enable real-time tracking/monitoring of infant's sleeping position, for example. Solution may be robust to the camera being placed at different locations and varying distances with respect to the crib.

Purely by way of example, one or more of the machine learning models may employ a Convolution Neural Network, because CNNs have been proven to be particularly successful at analysing images, and being able to identify objects within images, with a much lower error rate than other types of neural network.

In some embodiments, determining the infant's body posture may be further based on a history of one or more predicted body postures of the infant for a duration preceding the time of capture of the input image of the laying infant. For instance, a preceding body posture of the infant may be used to improve accuracy of the determination of the infant's current body posture.

An embodiment may further comprise: processing one or more historical input images of the laying infant with the object detection model to detect the infant's head and upper torso in each of the one or more historical input images; determining a second body axis of the laying infant based on the detected head and upper torso in each of the one or more historical input images, the body axis representing a longitudinal direction of the upper torso of the infant in each of the one or more historical input images; processing each of the one or more historical input images with the first keypoint detection model to detect and identify at least one shoulder of the infant in the one or more historical input images; determining the infant's body posture in each of the one or more historical input images based on the determined second body axis and the detected and identified shoulder(s) in the one or more historical input images; and predicting the infant's body posture based on: the determined infant's body posture in the input image or the outcome of a majority voting of the determined infant's body posture in each of the one or more historical input images.

Embodiments propose to determine the body posture of a laying infant from an input image or video of the laying infant. A hybrid AI concept may be employed to support analysis of the input image/video. The determined body posture outcomes may aid decision making and/or monitoring. Accordingly, embodiments may be used in relation to infant monitoring so as to support a parent or carer. For instance, embodiments may support the provision of a smart home care solution for the parent and child which may aid parents-in monitoring the sleep of their baby. The ability to monitor the sleep posture of an infant, potentially in real-time, may significantly reduce the risk(s) of SIDS (e.g. by alerting the parent(s) if the infant has rolled over to the on-belly position while sleeping).

In some embodiments, there is provided a computer program comprising instructions which, when the program is executed by a computer, cause the computer to carry out the method of any preceding claim.

According to another aspect of the invention, there is provided a processor arrangement configured to: process an input image of a laying infant with an object detection model to detect the infant's head and upper torso; determine a body axis based on the detected head and upper torso, wherein the body axis describes the longitudinal direction of the upper torso of the infant; process the input image with a first keypoint detection model to detect and identify at least one shoulder of the infant; determine an orientation of the identified shoulder(s) with respect to the body axis; and classify the infant's body posture into one of a plurality of body posture classifications based on the determined orientation of the identified shoulder(s) relative to the body axis.

According to another aspect of the invention, there is provided a system for determining the body posture of a laying infant according to claim 14.

In some embodiments, the system or processor arrangement described above may further comprise a user interface configured to receive, from the system or processor arrangement, and display an indication of the determined body posture of the laying infant.

The system may be remotely located from a user device for monitoring an infant. In this way, a user (such as a parent or carer) may have an appropriately arranged system that can receive information at a location remotely located from the system for determining the body posture of a laying infant. Embodiments may therefore enable a user to identify and monitor the body posture of a laying infant using a local system (which may, for example, comprise a portable display device, such as a laptop, tablet computer, mobile phone, PDA, etc.). By way of example, embodiments may provide an application for a mobile computing device, and the application may be executed and/or controlled by a user of the mobile computing device.

The system may further include: a server device comprising the system for determining the body posture of a laying infant; and a client device comprising a user-interface. Dedicated data processing means may therefore be employed for the purpose of determining the body posture of a laying infant, thus reducing processing requirements or capabilities of other components or devices of the system.

The system may further include a client device, wherein the client device comprises the image processing unit and a display unit. In other words, a user (such as a parent or carer) may have an appropriately arranged client device (such as a laptop, tablet computer, mobile phone, PDA, etc.) which processes received image data in order to determine the body posture of a laying infant and generate a display control signal. Purely by way of example, embodiments may therefore provide a monitoring or observation system that enables monitoring of one or more infants from a single location, wherein real-time communication between an infant and monitoring user (e.g. parent or carer) is provided and can have its functionality extended or modified according to proposed concepts, for example.

It will be understood that processing capabilities may therefore be distributed throughout the system in different ways according to predetermined constraints and/or availability of processing resources.

Thus, another aspect of the invention may provide an infant monitoring system that comprises: a system for determining the body posture of a laying infant according to a proposed embodiment; and an output interface configured to output an indication of the determined body posture of the infant.

Embodiments may be employed in combination with conventional/existing infant monitoring methods and systems. In this way, embodiments may integrate into legacy systems to improve and/or extend their functionality and capabilities. An improved infant monitoring system may therefore be provided by proposed embodiments.

Thus, there may be proposed concepts for determining the body posture of a laying infant, and this may be done based on the processing of an input image of the laying infant. These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Figs. 1A and 1B illustrate a first and second captured images of infant laying in first and second postures, respectively, wherein the head and upper body regions of the infant are identified by bounding boxes according to a proposed embodiment;
Figs. 2A and 2B illustrate the first and second captured images of Figs. 1A and 1B, respectively, wherein the head and upper body keypoints of the infant are identified by dots labelled "50";
Figs. 3A and 3B illustrate the first and second captured images of Figs. 1A and 1B, respectively, wherein the head and upper body keypoints of the infant are identified, and wherein the line segments connecting the head and upper body COMs are depicted;
Figs. 4A-4C illustrate different viewpoints of infant sleep posture with respect to a camera
Figs. 5A-5C illustrate flow diagrams to depicting an exemplary process for predicting body posture of a laying infant according to an embodiment;
Fig. 6 depicts a simplified flow diagram of a method for determining the body posture of a laying infant according to a proposed embodiment;
Fig. 7 is a simplified block diagram of a system for determining the body posture of a laying infant according to a proposed embodiment; and
Fig. 8 illustrates an example of a computer within which one or more parts of an embodiment may be employed.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

Variations to the disclosed embodiments can be understood and effected by those skilled in the art of practicing the claimed invention, from a study of the drawings, the disclosure, and the appended claims. In the claims, the word "comprising" does not exclude other elements or steps, and the indefinite article "a" or "an" does not exclude a plurality.

Implementations in accordance with the present disclosure relate to various techniques, methods, schemes and/or solutions pertaining to detecting and identifying the body posture of a laying infant (such as an infant sleeping in a cot/crib for example).

Embodiments of the invention aim to provide a concept for automatically determining the body posture of a laying infant. This can be achieved by automatically processing a captured image (or frame of a captured video) of a laying infant. In particular, it is proposed to analyse an input image of a laying infant to identify: (i) the longitudinal direction of the upper torso of the infant (i.e. body axis); and (ii) at least one shoulder of the infant. By analysing the orientation of the detected and identified shoulder(s) relative to the direction of the infant's upper torso, the infant's body posture may be determined. That is, it is proposed that the infant/s laying posture may be identified by analysing the orientation of the infant's shoulder(s) relative to the infant's body axis (in a captured image of the laying infant). Such a proposed approach may be simple but robust to the positioning of the image capture device. Also, the image may be obtained using widespread and prevalent/commonly-used devices that have image/video capture capabilities (such an infant monitor, webcam, or smartphone for example).

Proposed concepts thus aim to obtain a prediction of the body posture of a laying infant by use of image data alone. This may permit, for instance, the laying position of a sleeping infant to be automatically identified without the use of any special hardware, e.g. with only a smartphone or camera-equipped tablet computer. The automatically detected body posture may also be communicated to a user and/or stored in a memory, e.g. in a memory of the smartphone without the need of any manual intervention.

This invention may be of particular use in the case of monitoring a sleeping infant or baby. It is quite common for an infant to change orientation/position and/or roll over from back to lying on the belly and the vice versa during sleep. It is also common for a sleeping infant to be at partially covered by one or more blankets. A method capable of detecting a body posture of a laying infant which is agnostic to image capture position/viewpoint (e.g. position of camera device relative to the infant) and/or robust to infant coverage/occlusion by material/blankets would therefore be of great use.

Proposed embodiments may be based on computer vision and rule-based algorithms to determine the sleep posture of an infant in video recordings. These may be independent of the camera viewpoint and the camera angle. By way of example, a camera-based baby monitor can generate a live stream of sleeping (or laying) infant which is then fed to an AI model (hosted either in the Cloud or in processor arrangement). The AI model processes and analyses a video frame (i.e. a single image) to predict the body posture of the infant in the video frame, and this may be repeated regularly (e.g. every two-three seconds).

By way of summary, an exemplary embodiment comprises the following modules:
A. Image Augmentation: Each frame of an infant sleep dataset is augmented with random rotations and translations. Furthermore, to improve the robustness of the AI models for detecting the infant during night or in dim background lighting conditions, the brightness of RGB images is modified to mimic night vision and low light environments;
B. Object Detection: A pretrained lightweight object detection model is fine-tuned/trained on a database of video frames to detect the infant head and upper body in each frame. The head and upper body region of interests (ROI) are fed as input to a keypoint detection model;
C. Keypoint Detection: A pretrained keypoint detection model is fine-tuned/trained on a database of video frames for detecting keypoints in the infant head and upper body ROIs;
D. Sleep Posture Logic: Based on head and upper body ROIs and the predicted keypoints, sleep posture logic is employed to predict the infant sleep position (i.e. body posture). The sleep position may be classified from a set of predetermined classes (e.g. back, side and belly); and
E. Sleep Dashboard: A smart infant sleep dashboard is proposed for sending notifications. By way of example, notifications may inform a user whether the monitored infant is present in cot/crib, indicate a current sleep posture and recommend appropriate actions based on infant sleep patterns.

Considerations and exemplary implementation details of the abovementioned modules will now be described in the following paragraphs.

### - Image Dataset and Image Augmentation

An embodiment may fine-tune conventional AI models using an infant sleep dataset for predicting infant sleep posture. An example of a known/existing dataset comprises four hundred video images tracking the sleep position of several infants in real time. Each video is approximately 60 to 90 seconds in duration and the frame rate (i.e. number of frames per second) of each video is twenty-five. The sleep posture of twenty-two infants is tracked in the dataset and the maximum age of an infant is no more than one year. The subjects represented in the dataset are from countries with cold weather (indicated by the infant being covered with warm clothes and heavy blankets). Both RGB frames captured in daylight setting and grayscale frames representing night vision mode are available in the video dataset. If the infant is not able to fall asleep, then the infant will be moving or playing in the crib. Some of the challenges associated with the dataset may be as follows:
(i) The camera can be at any angle and any location with respect to laying infant;
(ii) The infant's body may be completely covered with heavy blankets making it difficult to identify the body of the infant;
(iii) The infant could be moving limbs frequently during sleep. Also, sometimes the hands completely cover the face making it difficult to detect the head.

The primary challenge with dataset is the sleep videos of a limited number of subjects are tracked in the dataset. The availability of a small number of subjects can impact the dataset distribution, which in turn can diminish the generalization performance of the AI models on new or previously unseen data. An approach in to improve the generalization performance of AI models is data augmentation. In computer vision, data augmentation comprises of a wide range of tools such as image translation, image rotation, brightness, and contrast alteration, blur etc. Embodiments may apply random shift(s) and random rotation(s) to images (i.e. video frames) in the dataset. Furthermore, transformations such as grayscale, gaussian blur, hue and saturation, random contrast, flips, and zooms may be applied to improve the diversity of the dataset.

### - Infant head and upper body detection

Object detection is a computer vision and image processing task where the aim is to detect instances of objects of a certain class in an image or a video. It is widely used in numerous computer vision applications such as vehicle detection, human and animal detection, facial recognition, real-time tracking the movements of vehicles and humans in video surveillance tasks. A popular AI model for object detection is You Only Look Once (YOLO). YOLO has achieved state-of-the-art performance on benchmark object detection datasets such as MS-COCO, Pascal VOC, and Cityscapes.

The task of an AI model for analysing an image of laying infant is to detect the head and upper body region of interests (ROIs) in the frame. The head region comprises the entire region at the top of the body including the face. The upper body comprises the head, upper limbs and extends up to the waist of a human. Here, it is noted that the human body parts below the waist are not included in the upper body.

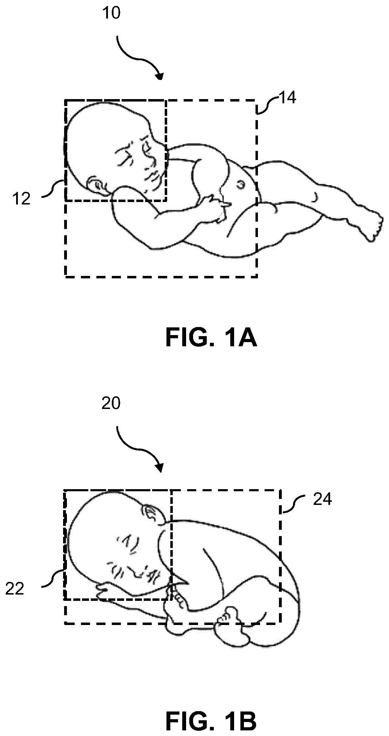

Figs. 1A and 1B illustrate a first and second captured images of an infant laying in first and second postures, respectively, wherein the head and upper body regions of the infant are identified by bounding boxes according to a proposed embodiment.

In Fig. 1A, the infant 10 is laying on its back. The head region is identified by a dashed bounding box labelled "12", and the upper body region is identified by a larger dashed bounding box labelled "14".

In Fig. 1B, the infant 20 is laying on its side. The head region is identified by a dashed bounding box labelled "22", and the upper body region is identified by a larger dashed bounding box labelled "24".

The first step is to annotate the region of interests (ROIs) in each video frame. Open-source annotation packages are widely available and may be used for annotating the ROIs with a box in each video frame of the image dataset.

Next, the dataset is divided into training and validation sets. The size of the training set may be further increased with image augmentation. Image augmentation can also address overfitting of an AI model.

An augmented training dataset may then be prepared for fine-tuning a pre-trained YOLO model for learning the bounding box of each ROI in a video frame. YOLO models can be both lightweight (e.g. low memory footprint) and heavyweight (e.g. high memory footprint) depending on the model requirements of an AI use case. Since the primary aim is to predict the ROIs with a low latency, a lightweight YOLO model may be fine-tuned on the augmented dataset and used to validate the AI model performance on the validation set. The fine-tuned YOLO model achieves a best mAP (mean Average Precision) score of 0.65 on the validation ROI dataset for detecting head and upper body ROIs. The AI model can also correctly predict when the infant is not present in the crib.

### - Infant keypoint detection

Keypoint detection is a computer vision task of detecting an object and localizing the keypoints of interest in the object. The object can be either animate including humans or inanimate such as vehicles. For example, the keypoints of interest in a human body could be eyes, ears, nose, mouth, shoulders, elbows, hands, hips, knees, and feet. One of the first keypoint detection areas of research in computer vision is human pose estimation where the problem is to accurately detect the keypoints of interest in a human body. Similarly, there have been a lot of interest in facial landmarks detection.

A known AI model for keypoint detection is the OpenPose model, which has achieved state-of-the-art performance on benchmark keypoint detection datasets such as MS-COCO keypoints, MPII human pose and VGG Pose datasets.

Typically, there are two types of keypoint annotation: top-down and bottom-up. Top-down AI model detects human candidates present in an image and then the keypoints corresponding to each candidate. Bottom-up models work the exact opposite of top-down models. These models first detect the keypoints present in an image and then the human candidates corresponding to the predicted keypoints.

Proposed embodiments may leverage a top-down human pose detection approach, wherein the first step involves detecting the infant upper body in a video frame, and then the head and upper body keypoints are predicted in the next step.

The task of a pose detection model for analysing a video frame of a laying infant is to detect the infant head and upper body keypoints in the frame. Keypoints in the head region comprises left ear, right ear, left eye, right eye, nose, and chin. Keypoints in the upper body comprises of left shoulder, right shoulder, left elbow, right elbow, left hand and right hand.

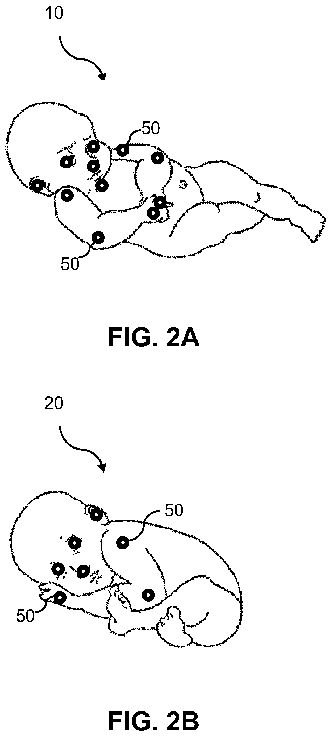

By way of example, Figs. 2A and 2B illustrate the first and second captured images of Figs. 1A and 1B, respectively, wherein the head and upper body keypoints of the infant are identified by dots labelled "50".

The first step is to annotate the keypoints of interests in each video frame. Open-source annotation packages are widely known and can be used for annotating the keypoints in each video frame of the dataset. Next, the image dataset is divided into training and validation sets. The size of the training set may be further increased with image augmentation to improve the generalizability of the AI models.

The augmented MCC training dataset may be prepared for fine-tuning a pretrained model for learning the keypoints in the upper body ROI in a video frame. The model can be both lightweight and heavyweight depending on the model requirements of an AI use case. Since the primary aim is to predict the keypoints with a low latency, a lightweight model may be preferred and fine-tuned on the augmented dataset and validate the AI model performance on the validation set. A fine-tuned model has been shown to achieve a best PCK (Percentage of Correct Keypoints) score of 0.84 on the validation dataset for detecting head and upper body keypoints. If the infant is not present in the crib, then the keypoints are not detected in the frame.

### - Sleep Posture Logic

The main objective of this section is developing a logic to accurately determine the sleep position - back, belly or side - based on predicted head and upper body region of interests and the corresponding keypoints of an infant. A proposed sleep posture algorithm leverages the head and upper body bounding box (e.g. bounding boxes in Figs. 1A and 1B) center of mass (COM) co-ordinates, the keypoint co-ordinates and elementary geometry.

As discussed in the above sections, the boxes are predicted by an object detection AI model and the keypoints are detected by a human pose estimation model.

For sleep posture algorithm, the left eye, right eye, left shoulder, right shoulder, left hand and right hand may be utilised. Note that if eyes are not visible in a frame, then the corresponding keypoints are not detected. However, the shoulder, elbow, and hand keypoints are detected even when they are occluded in a frame.

A line segment (AB) connects the head and upper body COM co-ordinates such that the head COM is closer to A. This line segment connecting the COMs of the head and upper body regions is used to define the 'body axis' of the infant (i.e. a line representing the longitudinal direction of the upper torso of the infant).

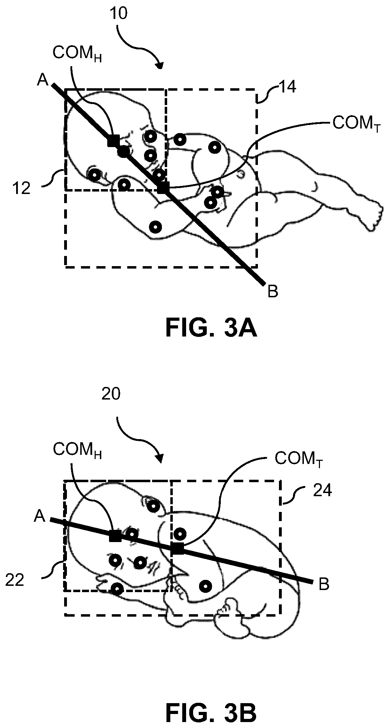

By way of example, Figs. 3A and 3B illustrate the first and second captured images of Figs. 1A and 1B, respectively, wherein the head and upper body keypoints of the infant are identified by dots labelled "50", and wherein the line segments (AB) connecting the head (COM_{H}) and upper body (COM_{T}) COMs are depicted. That is, Figs. 3A and 3B show how the line segments (AB) are derived from the COMs of the bounding boxes of the identified head and upper body, COM_{H} and COM_{T}. The line segment AB of each image represents the longitudinal direction of the torso of the infant, and is referred to as the body axis AB. The body axis may therefore be used to represent the general direction and orientation of the infant's torso.

It is proposed that, when an infant is sleeping on back posture, the keypoints in the left arm such as left shoulder and left hand are on the right side of the body axis AB, whereas the right arm keypoints are on the left side. The opposite holds true when an infant is lying on its belly. Further, it is proposed that the side sleep posture can be identified when both shoulders and both hands are on either side of the body axis AB.

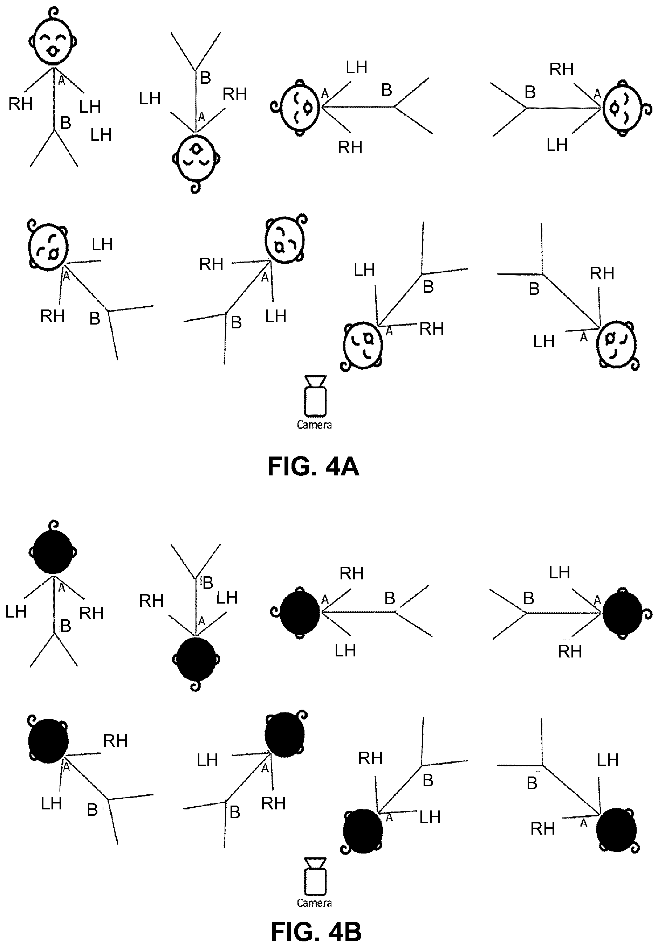

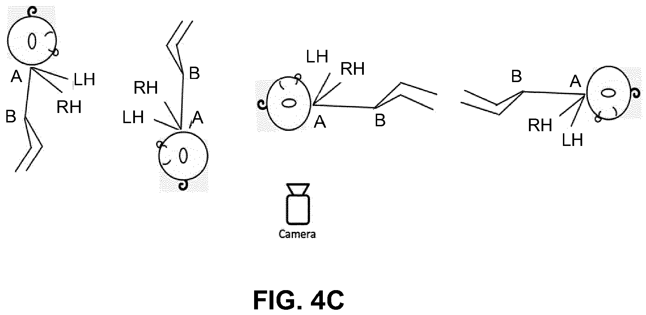

By way of example, Figs. 4A-4C illustrate different viewpoints of infant sleep posture with respect to a camera. The sleep postures - back, belly and side - are illustrated with varying degrees of camera viewpoint of an infant. From the illustrations of Fig. 4A, it can be seen that, when an infant is laying on its back (i.e. in a 'back' posture), the keypoints in the left arm, such as left shoulder and left hand, are on the right side of the body axis AB (from the viewpoint of the image), whereas the right arm keypoints are on the left side of the body axis AB (when viewed in from the viewpoint of the image). Conversely, and as illustrated in Fig. 4B, it can be seen that, when an infant is laying on its belly (i.e. in a 'belly' posture), the keypoints the left arm, such as left shoulder and left hand, are on the left side of the body axis AB (from the viewpoint of the image), whereas the right arm keypoints are on the right side of the body axis AB (when viewed in from the viewpoint of the image). Further, from the illustrations of Fig. 4C, it can be seen that, when an infant is laying on its side (i.e. in a 'side' posture), the keypoints in the left and right arms are on the same side of the body axis AB (from the viewpoint of the image).

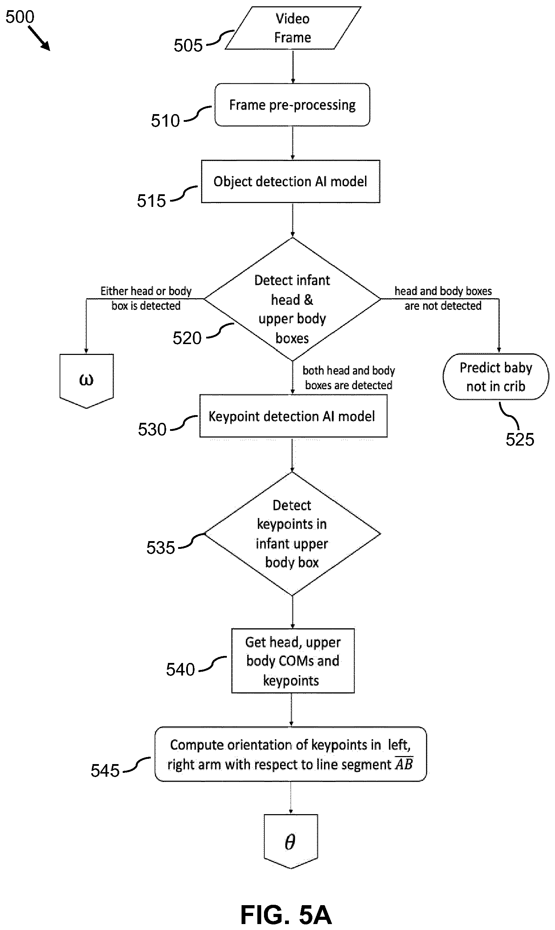

Bearing in mind the preceding description, and referring now to Figs. 5A-5C, there are depicted flow diagrams of a method for determining body posture of a laying infant according to an embodiment.

Referring now to Figs. 5A-5C, flow diagrams are illustrated to depict an exemplary high-level process for predicting body posture of a laying infant from an image 505 of laying infant (e.g. a frame 505 of a captured video). The flow diagrams include steps where AI models predict if an infant is present in the captured image (e.g. whether the infant is in the crib/cot), detecting the relevant boxes and keypoints, and finally predicting the body posture of the laying infant.

Firstly, in 510 the video frame is pre-processed to aid object detection. Such pre-processing may comprise, for example, comprise adjusting contrast, adjusting brightness, noise removal, etc. After pre-processing 510, the free- processed image is provided to an object detection AI model 515. Using the object detection AI model 515, the location of infant head and upper body bounding boxes is predicted in step 520.

If no head and upper body features are identified in step 520, it is determined in Step 525 that there is no infant present in the image (e.g.no infant in the crib/cot). Conversely, if head and upper body bounding boxes are determined in step 520, the method proceeds to step 530 wherein the video frame is provided to a keypoint detection AI model 530. If either head or upper body bounding box is determined in step 520, then the method proceeds to steps 585 and 595 wherein the video frame is provided to a facial keypoint detection AI model in step 585 and prediction of the sleep posture is based on one or more historical predictions in step 595.

Using the keypoint detection AI model 530, the location of keypoints are determined in step 540. Further, the location of head and upper body COMs is also determined in step 540. Based on the COMs, the body axis AB is also determined (e.g. defined by a line passing through the head and upper body COMs), wherein an orientation of the body axis AB relative to the viewing direction (i.e. viewpoint) of the image may be inferred/determined from the head to upper body direction. For instance, the orientation of body axis may be defined with respect to a direction of travel along axis from the head to the upper body. In this way, the body axis may represent a longitudinal direction of the upper torso of the infant and also the orientation of the upper torso with respect to the viewpoint of the image

The orientation of the keypoints in the left and right arms of the infant are computed with respect to the body axis AB in step 545.

The computed orientation of the shoulder keypoints in the left and right arms of the infant with respect to the body axis AB of the infant is checked in step 550.

If the shoulders are determined to be on the opposite side of the body axis AB, the orientation of the hands with respect to the body axis is then also checked in step 555. If they are on the same side, it is predicted in step 565 that the infant is laying on its side (i.e. in a 'side' body posture). However, if they are on the opposite side, the body posture of the infant is determined, in step 560, to be back or belly depending on the orientation of the shoulder and hand keypoints with respect to the body axis AB.

Conversely, if in step 550 it is determined that the shoulders are on the same side of the body axis AB, the orientation of the hands with respect to the body axis is then checked in step 570. If they are on the same side, it is predicted in step 575 that the infant is laying on its side (i.e. in a 'side' body posture). However, if they are on the opposite side, the body posture of the infant is determined, in step 580, to be 'back' or 'belly' depending on the orientation of the hand keypoints with respect to the body axis AB.

If only head bounding box is detected in step 520, the video frame in fed to a facial keypoint detection AI model in step 585. The location of the facial keypoints in the infant head bounding box is checked in step 590. If facial keypoints are not detected in step 590, then it is predicted in step 595 that the current prediction of the sleep posture is based on one or more historical predictions.

If facial keypoints are detected, it is then checked in step 600 if the detected keypoints include both eyes of the infant (i.e. checked whether the eyes are both visible) or the nose. If both eyes are visible, it is determined in step 605 that the infant is laying on its back (i.e. in a 'back' body posture). Conversely, if both eyes are not visible, , it is determined in step 610 that the infant is laying on its belly (i.e. in a 'belly' body posture).

Thus, given access to an image of a laying infant in a video frame, the following steps may be implemented to determine the body posture of the laying:
(i) Process an input image from a video stream of a laying infant with an object detection model to detect the infant's head and upper torso and a first keypoint detection model to identify and detect at least one shoulder of the infant.
(ii) If either head or upper body is detected, then it is determined that infant is present in cot/crib. If only head is detected, then go to step (iv-e). If only upper body is detected, then proceed to step (v).
(iii) If both head and upper body are undetected, then it is determined that infant is not present in cot/crib. Proceed to step (vi).
(iv) If both head and upper body are detected, then it is determined that infant is present in cot/crib.
   a. Determine the body axis of the infant from the detected head and upper torso.
   b. If at least one shoulder of infant is detected, then determine the body posture in the first input image based on the determined body axis and the orientation of the detected and identified shoulder(s) relative to the body axis.
   c. Further, if at least one hand is detected, then determine the body posture in the first input image based on the determined body axis and the orientation of the detected and identified hand(s) relative to the body axis.
   d. If neither shoulder nor hand is detected, then process the input image with a second keypoint detection model to detect one or more facial keypoints (e.g. eyes, nose) of the infant.
   e. If facial keypoints are detected by the second keypoint detection model, then determine infant's body posture based on the detected one or more facial keypoints.
   f. If one or more facial keypoints are not detected, then proceed to step (v). Else proceed to step (vi).
(v). Determine the infant's latest body posture based on a recent history of one or more predicted body postures of the infant identified for a duration preceding the time of capture of the latest input image of the infant in bed. Predict the infant's latest body posture based on the outcome of a majority voting of the determined infant's body posture in each of the input image(s) of the recent history.
(vi) Repeat the above steps for subsequent input images from video stream of laying infant.

The proposed algorithm may work accurately when an infant is sleeping in a crib/cot and the image capture device is placed near the infant. However, when the infant rolls over from a current sleep posture during sleep, the movements might lead to erroneous AI model prediction in a few frames, but then the predictions may become stable and accurate once the infant settles down in a new posture.

There could be exceptions to the above sleep posture logic. For example, an infant could be looking at the camera while lying on the belly. Such instances are not considered in the domain set which includes infant sleeping soundly in crib.

### - Infant sleep monitor and dashboard

An infant sleep monitor and a dashboard is also proposed to aid the parents in monitoring the sleep of their infant in real time.

The dashboard may comprise a user interface that includes a live video of an infant lying in the crib. The video streaming can be turned on or off depending on the requirements of the parents and the resources available in the mobile device. Several features related to different aspects of infant sleep may be tracked and communicated by the dashboard in real time such as: whether infant is present in crib; infant awake or asleep status; infant breathing status (if yes, an indication of whether breathing is within normal range may also be provided); an indication of whether the infant's face (particularly the nose) is covered with any object; an indication of the infants current body posture; and indication of the time spent lying on the current body posture since moving from the previous sleep posture; and audible and/or visible alert(s) if the infant rolls over to belly posture and/or when there is an anomaly in breathing rate.

The dashboard may thus provide high-level analytics/insights regarding the infant's sleep habits (based on the sleep history for example). For example, a bar chart of total sleep duration in past seven days may be displayed to a user. Such data can support the parents in determining if the sleep cycle of the infant is being affected when there is an anomaly in the chart. The bar chart may also extend to providing the sleep duration in past two weeks or past month. The dashboard may also display a pie chart conveying how much time an infant spends sleeping on a particular posture and how it compares with the rest of the postures. This can aid the parent in identifying which sleep posture is an infant comfortable with during sleep.

Sleep status reports describing sleep data (such as the average, maximum and minimum number of hours an infant sleep daily, etc.) may also be provided by the dashboard. Furthermore, a sleep interruption report may also be provided which includes information on whether any interruption occurred during sleep. The report may track the average number of interruptions occurring daily during sleep, when does most sleep interruptions happen - daytime or at night, and the potential cause of sleep interruption - external noise, abnormal breathing, crying, etc. Also, a sleep ambient report may be provided which includes information describing the average temperature and humidity of the room where the infant lies in the crib. Finally, a breathing rate report may be provided which tracks the breathing activity of the infant in real time and an alert may be triggered to the parents when an anomaly in infant breathing is detected.

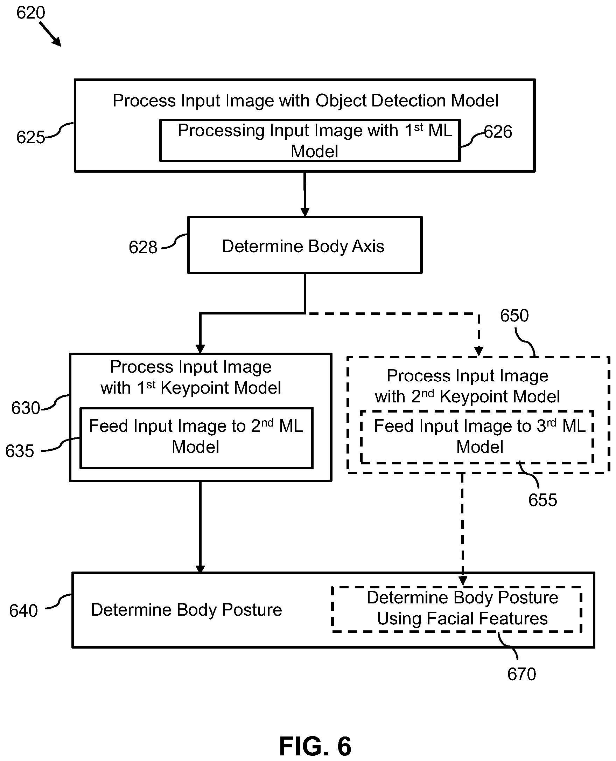

By way of yet further explanation of the proposed concept(s), and referring now to Fig. 6, there is depicted a flow diagram of a method 620 for determining the body posture of a laying infant according to a proposed embodiment.

The method begins with the step 625 of processing an input image of the laying infant with an object detection model to detect the infant's head and upper torso. The input image can be obtained using existing or conventional devices that include cameras already owned by a user. For instance, a conventional device may be one of: a smartphone, a tablet, a laptop, or any other suitable device.

Here, the step 625 of processing the input image of the infant with the object detection model comprises: feeding 626 the input image to a first machine learning model. The first machine learning mode comprises a convolutional neural network, CNN, the CNN being trained to predict co-ordinates of bounding boxes representing the spatial location of the infant's head and upper torso in the input image. CNNs have been proved to be particularly successful at analysing images and being able to identify objects within images with a much lower error rate than other types of neural network.

The structure of an artificial neural network (or, simply, neural network) is inspired by the human brain. Neural networks are comprised of layers, each layer comprising a plurality of neurons. Each neuron comprises a mathematical operation. In particular, each neuron may comprise a different weighted combination of a single type of transformation (e.g. the same type of transformation, sigmoid etc. but with different weightings). In the process of processing input data, the mathematical operation of each neuron is performed on the input data to produce a numerical output, and the outputs of each layer in the neural network are fed into the next layer sequentially. The final layer provides the output.

There are several types of neural network, such as convolutional neural networks (CNNs) and recurrent neural networks (RNNs). This exemplary embodiment of the present invention employs CNN-based learning algorithms, because CNNs have proved to be particularly successful at analyzing images, and are able to identify objects within images with a much lower error rate than other types of neural network.

CNNs typically contain several layers, including a convolutional layer, a pooling layer, and a fully connected layer. The convolutional layer consists of a set of learnable filters and extracts features from the input. The pooling layer is a form of non-linear down-sampling, reducing the data size by combining the outputs of a plurality of neurons in one layer into a single neuron in the next layer. The fully connected layer connects each neuron in one layer to all the neurons in the next layer.

Methods of training a machine-learning algorithm are well known. Typically, such methods comprise obtaining a training dataset, comprising training input data entries and corresponding training output data entries. An initialized machine-learning algorithm is applied to each input data entry to generate predicted output data entries. An error between the predicted output data entries and corresponding training output data entries is used to modify the machine-learning algorithm. This process can be repeated until the error converges, and the predicted output data entries are sufficiently similar (e.g. ±1%) to the training output data entries. This is commonly known as a supervised learning technique.

For example, weightings of the mathematical operation of each neuron may be modified until the error converges. Known methods of modifying a neural network include gradient descent, backpropagation algorithms and so on.

The training input data entries for the first machine learning model which may be used in step 626 correspond to example images of an infant laying or sleeping in a bed. The training output data entries correspond to the co-ordinates of the bounding boxes representing the spatial location of the infant's head and upper torso in the training image. Further, several pre-processing methods may be employed to improve the training samples. In other words, the first CNN may be trained using a using a deep learning algorithm configured to receive an array of training inputs and respective known outputs, wherein a training input comprises a training image of an infant laying or sleeping in a bed and a respective known output comprises the co-ordinates of the bounding boxes representing the spatial location of the infant's head and upper torso in the training image. In this way, the CNN may be trained to output a prediction of the spatial location of the infant's head and upper torso when provided with an image of a lying infant.

The CNN may be a pre-trained model further trained on images of a laying or sleeping infant that have been manually annotated. For example, as previously discussed above, short videos may be taken of laying infants from various angles and different body postures. Images may then be extracted from the videos in order to serve as training inputs with known head and upper torso positions. This allows the CNN to become especially proficient at predicting the head and upper torso positions, regardless of image capture (i.e. camera) position.

Next, in step 628, a body axis of the infant is determined based on the detected head and upper torso, wherein the body axis represents a longitudinal direction of the upper torso of the infant. As explained with reference to previous embodiments, the body axis may be defined by a line passing through the COMs of the head and upper torso positions. However, embodiments need to be limited to employing the COMs of the head and upper torso positions. For instance, in this exemplary embodiment, the body axis may be determined to be a line dissecting an angle formed between the diagonal lines connecting opposing corners of the head and upper torso bounding boxes. In another example, the body axis may be defined based on a rotated version of diagonal line connecting opposing corners of the upper torso bounding box, wherein the angle of rotation is based on the angle of the diagonal line connecting opposing corners of the head bounding box.

In yet another example, the body axis may be determined based on eyes and shoulder/hands keypoints (without requiring a head bounding box). Specifically, the body axis may be defined by: determining the mid-point (E) of the line segment connecting both eye co-ordinates and the mid-point of the line segment connecting both shoulder (S) keypoints; and constructing a line segment passing from point E to point S. Such a line segment may define a body axis representing the longitudinal direction of the infant/subject body/upper torso.

The method then proceeds to the step 630 of processing the input image with a first keypoint detection model to detect and identify at least one shoulder of the infant. Here, processing the input image with the first keypoint detection model comprises the step 635 of feeding the input image to a second machine learning model. In this example, the second machine-learning model is trained to identify and predict the co-ordinates representing the spatial location of the infant's shoulder(s) and hand(s) in the input image. In particular, the second machine learning model is trained using a deep learning algorithm configured to receive an array of training inputs and respective known outputs, wherein a training input comprises of a training image of an infant laying or sleeping in a bed and a respective known output comprises of the co-ordinates of the infant's shoulder and hand keypoints in the training image.

Finally, in step 640, the infant's body posture in the input image is determined based on the determined body axis and the orientation of the detected and identified shoulder(s) relative to the body axis. Here, determining the infant's body posture comprises: determining an orientation of the identified shoulder(s) with respect to the body axis; and classifying the infant's body posture into one of a plurality of body posture classifications (such as back, belly or side) based on the determined orientation of the identified shoulder(s) relative to the body axis. For example, the logic of the example described above with reference to the illustrations of Figs. 4A-4C may be applied, so as to determine body posture classifications according to the orientation of the identified shoulder(s) relative to the body axis.

The determined body posture may be output from the method, for example as an output signal for monitoring system and/or as a display control signal for displaying information about the posture to a user.

Although the embodiment described above has been detailed as determining body posture classifications according to the orientation of the identified shoulder(s) relative to the body axis, other embodiments may also employ other detected keypoints to qualify and/or improve body posture determination.

Purely by way of example, the flow diagram of Fig. 6 depicts additional steps that may be employed in another embodiment, wherein the additional steps are indicated by dashed boxes. Specifically, an embodiment may further comprise the step 650 of processing the input image with a second keypoint detection model to detect one or more facial features of the infant.

Here, processing 650 the input image with the second keypoint detection model comprises feeding the input image to a third machine learning model, wherein the third machine learning model is trained to identify and predict the co-ordinates representing the spatial location of one or more facial features of the infant in the input image. More specifically, the third machine learning models is trained using a deep learning algorithm configured to receive an array of training inputs and respective known outputs, wherein a training input comprises of a training image of an infant lying down or sleeping in a bed and a respective known output comprises of the co-ordinates of the infant's facial feature keypoints such as eyes, nose, and ears in the training image.

The step 640 of determining the infant's body posture may comprise step 670 of determining the infant's body posture further based on the detected one or more facial features. For instance, step 670 of determining the infant's body posture in this example comprises: determining if the detected one or more facial features of the infant comprises eyes or nose of the infant; and classifying the infant's body posture into one of a plurality of body posture classifications responsive to determining the detected one or more facial features of the infant comprises eyes or nose of the infant.

Other variations and/or additions to the above-detailed embodiments may be employed. By way of example, any of the machine learning models may be further trained and fine-tuned based on manually annotated images of an infant lying down or sleeping in a bed. Also, determining the infant's body posture may be further based on a history of one or more predicted body postures of the infant for a duration preceding the time of capture of the input image of the laying infant. In this way, a preceding body posture of the infant (e.g. detected in preceding video frame) may be used to improve accuracy of the determination of the infant's current body posture.

In some embodiments, there is provided a computer program comprising code means for implementing any of the methods described above when said program is run on a processing system.

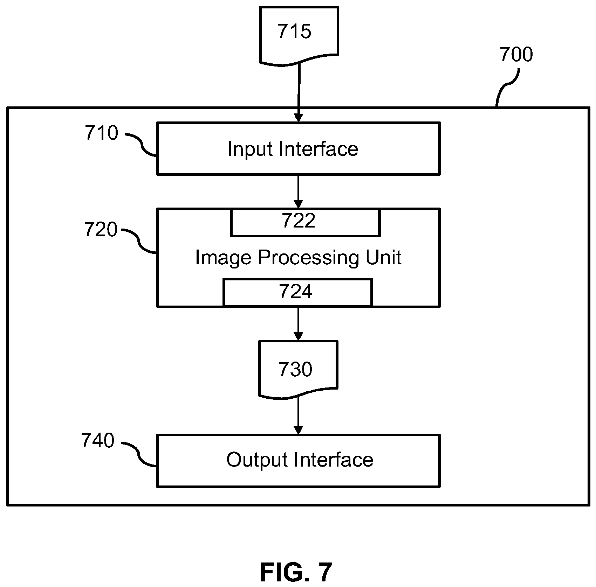

Referring now to Fig. 7, there is depicted a simplified block diagram of a system 700 for determining the body posture of a laying infant according to a proposed embodiment. The system configured for determining the body posture of a laying infant comprises an input interface 710, and an image processing unit 720. The image processing unit 720 may also be referred to as a processor arrangement.

The system 700 is configured to determine the body posture of a laying infant by processing an input image 715 of a laying infant. An input image 715 of an infant laying on a mattress or support surface infant is obtained. The system generates an identification 730 of the infant's body posture. The identification 730 of the infant's body posture is determined based on processing of the input image in order to determine the infant's body posture in the input image based on the orientation of the detected and identified shoulder(s) relative to the body axis.

In more detail, the input interface 710 receives an input image 715 of an infant laying on a mattress or support surface infant is obtained. The input interface 710 provides the input image 715 to an image processing unit 720 (via an input port/interface 722 of the image processing unit 720). The image processing unit 720 is configured to process the input image 715 with an object detection model to detect the infant's head and upper torso. A body axis is then determined based on the detected head and upper torso, the body axis representing a longitudinal direction of the upper torso of the infant. The image processing unit 720 also processes the input image with a first keypoint detection model to detect and identify at least one shoulder of the infant. The image processing unit then determines the infant's body posture in the input image based on the determined body axis and the orientation of the detected and identified shoulder(s) relative to the body axis. The image processing unit 720 is configured to output identification 730 of the infant's body posture via an output port/interface 724 of the image processing unit 720.

An output interface 740 is configured to receive (from output port of the image processing unit 720) the identification 730 of the infant's body posture and output it (e.g. as an electronic output signal and/or a visible/audible output).

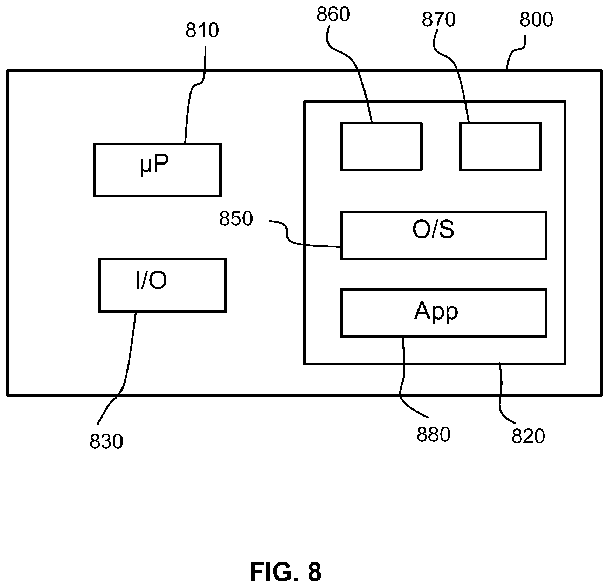

Fig. 8 illustrates an example of a computer 800 within which one or more parts of an embodiment may be employed. Various operations discussed above may utilize the capabilities of the computer 800. In this regard, it is to be understood that system functional blocks can run on a single computer or may be distributed over several computers and locations (e.g. connected via internet).

The computer 800 includes, but is not limited to, PCs, workstations, laptops, PDAs, palm devices, servers, storages, and the like. Generally, in terms of hardware architecture, the computer 800 may include one or more processors 810, memory 820 and one or more I/O devices 830 that are communicatively coupled via a local interface (not shown). The local interface can be, for example but not limited to, one or more buses or other wired or wireless connections, as is known in the art. The local interface may have additional elements, such as controllers, buffers (caches), drivers, repeaters, and receivers, to enable communications. Further, the local interface may include address, control, and/or data connections to enable appropriate communications among the aforementioned components.

The processor 810 is a hardware device for executing software that can be stored in the memory 820. The processor 810 can be virtually any custom made or commercially available processor, a central processing unit (CPU), a digital signal processor (DSP), or an auxiliary processor among several processors associated with the computer 800, and the processor 810 may be a semiconductor-based microprocessor (in the form of a microchip) or a microprocessor.

The memory 820 can include any one or combination of volatile memory elements (e.g., random access memory (RAM), such as dynamic random access memory (DRAM), static random access memory (SRAM), etc.) and non-volatile memory elements (e.g., ROM, erasable programmable read only memory (EPROM), electronically erasable programmable read only memory (EEPROM), programmable read only memory (PROM), tape, compact disc read only memory (CD-ROM), disk, diskette, cartridge, cassette or the like, etc.). Moreover, the memory 820 may incorporate electronic, magnetic, optical, and/or other types of storage media. Note that the memory 820 can have a distributed architecture, where various components are situated remote from one another, but can be accessed by the processor 810.

The software in the memory 820 may include one or more separate programs, each of which comprises an ordered listing of executable instructions for implementing logical functions. The software in the memory 820 includes a suitable operating system (O/S) 850, compiler 860, source code 870, and one or more applications 880 in accordance with exemplary embodiments. As illustrated, the application 880 comprises numerous functional components for implementing the features and operations of the exemplary embodiments. The application 880 of the computer 800 may represent various applications, computational units, logic, functional units, processes, operations, virtual entities, and/or modules in accordance with exemplary embodiments, but the application 880 is not meant to be a limitation.

The operating system 850 controls the execution of other computer programs, and provides scheduling, input-output control, file and data management, memory management, and communication control and related services. It is contemplated by the inventors that the application 880 for implementing exemplary embodiments may be applicable on all commercially available operating systems.

Application 880 may be a source program, executable program (object code), script, or any other entity comprising a set of instructions to be performed. When a source program, then the program is usually translated via a compiler (such as the compiler 860), assembler, interpreter, or the like, which may or may not be included within the memory 820, so as to operate properly in connection with the O/S 850. Furthermore, the application 880 can be written as an object oriented programming language, which has classes of data and methods, or a procedure programming language, which has routines, subroutines, and/or functions, for example but not limited to, C, C++, C#, Pascal, Python, BASIC, API calls, HTML, XHTML, XML, ASP scripts, JavaScript, FORTRAN, COBOL, Perl, Java, ADA, .NET, and the like.

The I/O devices 830 may include input devices such as, for example but not limited to, a mouse, keyboard, scanner, microphone, camera, etc. Furthermore, the I/O devices 830 may also include output devices, for example but not limited to a printer, display, etc. Finally, the I/O devices 830 may further include devices that communicate both inputs and outputs, for instance but not limited to, a NIC or modulator/demodulator (for accessing remote devices, other files, devices, systems, or a network), a radio frequency (RF) or other transceiver, a telephonic interface, a bridge, a router, etc. The I/O devices 830 also include components for communicating over various networks, such as the Internet or intranet.

If the computer 800 is a PC, workstation, intelligent device or the like, the software in the memory 820 may further include a basic input output system (BIOS) (omitted for simplicity). The BIOS is a set of essential software routines that initialize and test hardware at start-up, start the O/S 850, and support the transfer of data among the hardware devices. The BIOS is stored in some type of read-only-memory, such as ROM, PROM, EPROM, EEPROM or the like, so that the BIOS can be executed when the computer 800 is activated.

When the computer 800 is in operation, the processor 810 is configured to execute software stored within the memory 820, to communicate data to and from the memory 820, and to generally control operations of the computer 800 pursuant to the software. The application 580 and the O/S 850 are read, in whole or in part, by the processor 810, perhaps buffered within the processor 810, and then executed.

When the application 880 is implemented in software it should be noted that the application 880 can be stored on virtually any computer readable medium for use by or in connection with any computer related system or method. In the context of this document, a computer readable medium may be an electronic, magnetic, optical, or other physical device or means that can contain or store a computer program for use by or in connection with a computer related system or method.

The application 880 can be embodied in any computer-readable medium for use by or in connection with an instruction execution system, apparatus, or device, such as a computer-based system, processor-containing system, or other system that can fetch the instructions from the instruction execution system, apparatus, or device and execute the instructions. In the context of this document, a "computer-readable medium" can be any means that can store, communicate, propagate, or transport the program for use by or in connection with the instruction execution system, apparatus, or device. The computer readable medium can be, for example but not limited to, an electronic, magnetic, optical, electromagnetic, infrared, or semiconductor system, apparatus, device, or propagation medium.

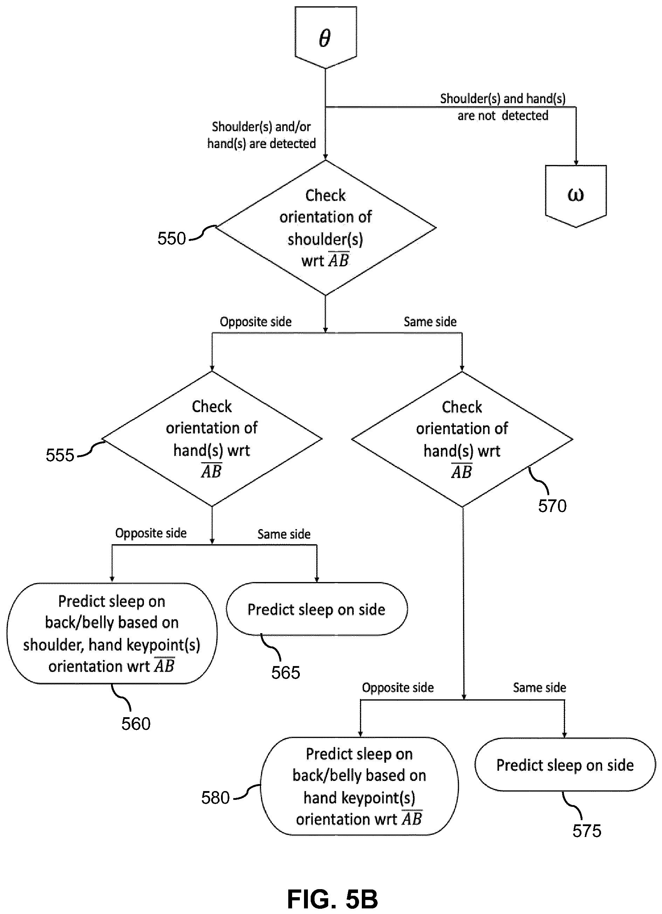

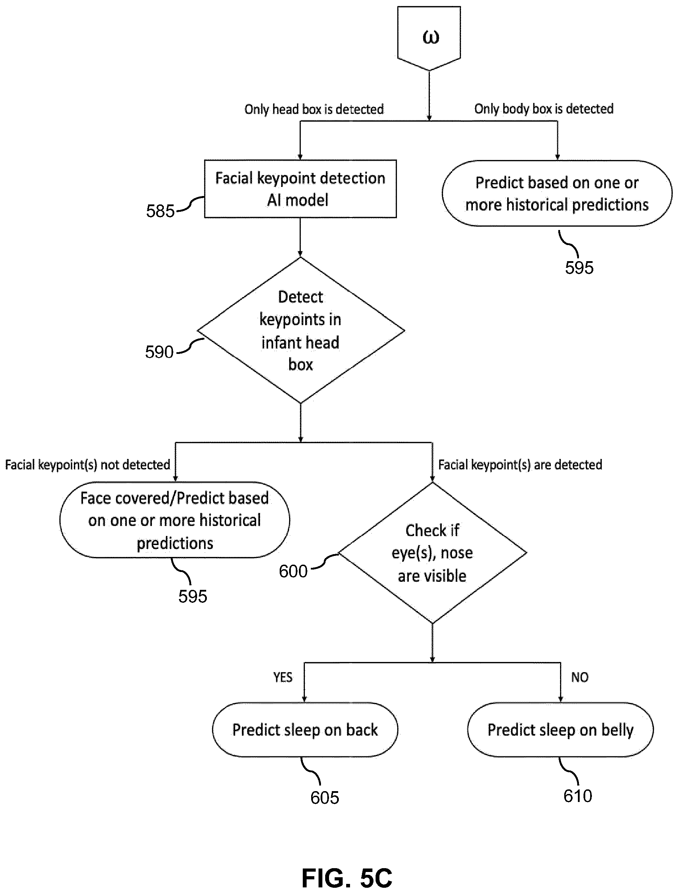

The methods of Figs. 5-6, and the system of Fig. 7, may be implemented in hardware or software, or a mixture of both (for example, as firmware running on a hardware device). To the extent that an embodiment is implemented partly or wholly in software, the functional steps illustrated in the process flowcharts may be performed by suitably programmed physical computing devices, such as one or more central processing units (CPUs) or graphics processing units (GPUs). Each process - and its individual component steps as illustrated in the flowcharts - may be performed by the same or different computing devices. According to embodiments, a computer-readable storage medium stores a computer program comprising computer program code configured to cause one or more physical computing devices to carry out a method as described above when the program is run on the one or more physical computing devices.

Storage media may include volatile and non-volatile computer memory such as RAM, PROM, EPROM, and EEPROM, optical discs (like CD, DVD, BD), magnetic storage media (like hard discs and tapes). Various storage media may be fixed within a computing device or may be transportable, such that the one or more programs stored thereon can be loaded into a processor.

To the extent that an embodiment is implemented partly or wholly in hardware, the blocks shown in the block diagrams of Fig. 7 may be separate physical components, or logical subdivisions of single physical components, or may be all implemented in an integrated manner in one physical component. The functions of one block shown in the drawings may be divided between multiple components in an implementation, or the functions of multiple blocks shown in the drawings may be combined in single components in an implementation. Hardware components suitable for use in embodiments of the present invention include, but are not limited to, conventional microprocessors, application specific integrated circuits (ASICs), and field-programmable gate arrays (FPGAs). One or more blocks may be implemented as a combination of dedicated hardware to perform some functions and one or more programmed microprocessors and associated circuitry to perform other functions.

Embodiments of the proposed invention may be employed to accurately determine the body posture of a laying infant (such as an infant sleeping in a crib) during the day and at night with minimal background lighting. The proposed model-based approach may also be robust to arbitrary camera viewpoints of the infant.

Embodiments may be employed in conjunction with frames of a livestream video, so as to detect and monitor an infant's body posture in real time. It may further generate one or more notifications or alerts responsive to the detected and monitored body posture of the infant.

It will therefore be appreciated from the above detailed description of various embodiments and proposed concepts that a simple yet effective hybrid AI method is proposed for accurate prediction of infant sleep posture. One such approach may comprise three main algorithms: a) object detection, b) keypoint detection, and c) sleep posture determination algorithm. Embodiments may leverages state-of-the-art AI models for object detection and keypoint detection tasks, and a concept for predicting infant sleep posture based on ROIs and keypoints may be employed.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality. A single processor or other unit may fulfil the functions of several items recited in the claims. The mere fact that certain measures are recited in mutually different dependent claims does not indicate that a combination of these measures cannot be used to advantage. If a computer program is discussed above, it may be stored/distributed on a suitable medium, such as an optical storage medium or a solid-state medium supplied together with or as part of other hardware, but may also be distributed in other forms, such as via the Internet or other wired or wireless telecommunication systems. If the term "adapted to" is used in the claims or description, it is noted the term "adapted to" is intended to be equivalent to the term "configured to". Any reference signs in the claims should not be construed as limiting the scope.

The flowchart and block diagrams in the Figures illustrate the architecture, functionality, and operation of possible implementations of systems, methods, and computer program products according to various embodiments of the present invention. In this regard, each block in the flowchart or block diagrams may represent a module, segment, or portion of instructions, which comprises one or more executable instructions for implementing the specified logical function(s). In some alternative implementations, the functions noted in the block may occur out of the order noted in the Figures. For example, two blocks shown in succession may, in fact, be executed substantially concurrently, or the blocks may sometimes be executed in the reverse order, depending upon the functionality involved. It will also be noted that each block of the block diagrams and/or flowchart illustration, and combinations of blocks in the block diagrams and/or flowchart illustration, can be implemented by special purpose hardware-based systems that perform the specified functions or acts or carry out combinations of special purpose hardware and computer instructions.the architecture, functionality, and operation of possible implementations of systems, methods, and computer program products according to various embodiments of the present invention. In this regard, each block in the flowchart or block diagrams may represent a module, segment, or portion of instructions, which comprises one or more executable instructions for implementing the specified logical function(s). In some alternative implementations, the functions noted in the block may occur out of the order noted in the Figures. For example, two blocks shown in succession may, in fact, be executed substantially concurrently, or the blocks may sometimes be executed in the reverse order, depending upon the functionality involved. It will also be noted that each block of the block diagrams and/or flowchart illustration, and combinations of blocks in the block diagrams and/or flowchart illustration, can be implemented by special purpose hardware-based systems that perform the specified functions or acts or carry out combinations of special purpose hardware and computer instructions.

## Claims

1. A method for determining the body posture of a laying infant, the method comprising:
processing (610) an input image of the laying infant with an object detection model to detect the infant's head and upper torso;
determining (620) a body axis based on the detected head and upper torso, wherein the body axis describes the longitudinal direction of the upper torso of the infant;
processing (630) the input image with a first keypoint detection model to detect and identify at least one shoulder of the infant;
determining an orientation of the identified shoulder(s) with respect to the body axis; and
classifying the infant's body posture into one of a plurality of body posture classifications based on the determined orientation of the identified shoulder(s) relative to the body axis.

2. The method of claim **1,** wherein processing an input image of the laying infant with an object detection model to detect infant's head and upper torso comprises:
processing the input image of the laying infant with an object detection model to generate a bounding box for each of the infant's head and upper torso respectively; and
wherein determining a body axis based on the detected head and upper torso comprises:
determining a body axis based on the generated boundary boxes, such that the body axis passes through the center of mass of each bounding box.

3. The method of claim 1 or 2, further comprising:
processing the input image with a second keypoint detection model to detect one or more facial features of the infant,
and wherein determining the infant's body posture is further based on the detected one or more facial features,
and optionally wherein determining the infant's body posture comprises:
determining if the detected one or more facial features of the infant comprises eyes or nose of the infant; and
classifying the infant's body posture into one of a plurality of body posture classifications responsive to determining the detected one or more facial features of the infant comprises eyes or nose of the infant.

4. The methods of any of claims 1 to 3, wherein determining the body axis comprises determining an orientation of the body axis, and wherein determining the infant's body posture is further based on the determined orientation of the body axis.

5. The method of any of claims 1 to 4, further comprising:
processing the input image with the first keypoint detection model to detect and identify at least one hand of the infant,
and wherein determining the infant's body posture is further based on the determined body axis and the orientation of detected and identified hand(s) relative to the body axis.

6. The method of any of claims 1 to 5, wherein processing an input image of the infant with the object detection model comprises:
feeding the input image to a first machine learning model wherein the first machine learning model is trained to predict co-ordinates of bounding boxes representing the spatial location of the infant's head and upper torso in the input image,
and preferably wherein the first machine learning model is trained using a deep learning algorithm configured to receive an array of training inputs and respective known outputs, wherein a training input comprises a training image of an infant laying or sleeping in a bed and a respective known output comprises the co-ordinates of the bounding boxes representing the spatial location of the infant's head and upper torso in the training image.

7. The method of any of claims 1 to 6, wherein processing the input image with the first keypoint detection model comprises:
feeding the input image to a second machine learning model, wherein the second machine-learning model is trained to identify and predict the co-ordinates representing the spatial location of the infant's shoulder(s) and hands in the input image,
and preferably wherein the second machine learning model is trained using a deep learning algorithm configured to receive an array of training inputs and respective known outputs, and wherein a training input comprises of a training image of an infant laying or sleeping in a bed and a respective known output comprises of the co-ordinates of the infant's shoulder and hand keypoints in the training image.

8. The method of any of claims 1 to 7, wherein processing the input image with the second keypoint detection model comprises:
feeding the input image to a third machine learning model wherein the third machine learning model is trained to identify and predict the co-ordinates representing the spatial location of one or more facial features of the infant in the input image,
and preferably wherein the third machine learning models is trained using a deep learning algorithm configured to receive an array of training inputs and respective known outputs., and wherein a training input comprises of a training image of an infant lying down or sleeping in a bed and a respective known output comprises of the co-ordinates of the infant's facial feature keypoints such as eyes, nose, and ears in the training image.

9. The method of claim 6, 7 or 8, wherein at least one of the first, second and third machine learning models comprises a pretrained model further trained and fine-tuned on manually annotated images of an infant lying down or sleeping in a bed.

10. The method of any of claims 1 to 9, wherein determining the infant's body posture is further based on a history of one or more predicted body postures of the infant for a duration preceding the time of capture of the input image of the laying infant.

11. The method of any of claims 1 to 10, further comprising:
processing a one or more historical input images of the laying infant with the object detection model to detect the infant's head and upper torso in each of the one or more historical input images;
determining a second body axis of the laying infant based on the detected head and upper torso in each of the one or more historical input images, the body axis representing a longitudinal direction of the upper torso of the infant in each of the one or more historical input images;
processing each of the one or more historical input images with the first keypoint detection algorithm model to detect and identify at least one shoulder of the infant in the one or more historical input images;
determining the infant's body posture in each of the one or more historical input images based on the determined second body axis and the detected and identified shoulder(s) in the one or more historical input images; and
predicting the infant's body posture based on: the determined infant's body posture in the input image or the outcome of a majority voting of the determined infant's body posture in each of the one or more historical input images.

12. A computer program comprising instructions which, when the program is executed by a computer, cause the computer to carry out the method of any preceding claim.

13. A processor arrangement configured to:
process an input image of a laying infant with an object detection model to detect the infant's head and upper torso;
determine a body axis based on the detected head and upper torso, wherein the body axis describes the longitudinal direction of the upper torso of the infant;
process the input image with a first keypoint detection model to detect and identify at least one shoulder of the infant;
determine an orientation of the identified shoulder(s) with respect to the body axis; and
classify the infant's body posture into one of a plurality of body posture classifications based on the determined orientation of the identified shoulder(s) relative to the body axis.

14. A system (700) for determining the body posture of a laying infant, the system comprising:
the processor arrangement of claim 13, wherein the processor arrangement is an image processing unit (720).

15. An infant monitoring system, comprising:
the system of claim 14; and
an output interface (740) configured to output an indication of the determined body posture of the infant.

## Patentansprüche

1. Verfahren zum Bestimmen der Körperhaltung eines liegenden Säuglings, wobei das Verfahren Folgendes umfasst:
Verarbeiten (610) eines Eingabebilds des liegenden Säuglings mit einem Objekterkennungsmodell, um den Kopf und Oberkörper des Säuglings zu erkennen;
Bestimmen (620) einer Körperachse basierend auf dem erkannten Kopf und Oberkörper, wobei die Körperachse die Längsrichtung des Oberkörpers des Säuglings beschreibt;
Verarbeiten (630) des Eingabebilds mit einem ersten Schlüsselpunkterkennungsmodell, um mindestens eine Schulter des Säuglings zu erkennen und zu identifizieren;
Bestimmen einer Ausrichtung der identifizierten Schulter(n) in Bezug auf die Körperachse; und
Klassifizieren der Körperhaltung des Säuglings in eine von einer Vielzahl von Körperhaltungsklassifizierungen basierend auf der bestimmten Orientierung der identifizierten Schulter(n) relativ zur Körperachse.

2. Verfahren nach Anspruch 1, wobei Verarbeiten eines Eingabebilds des liegenden Säuglings mit einem Objekterkennungsmodell, um den Kopf und Oberkörper des Säuglings zu erkennen, Folgendes umfasst:
Verarbeiten des Eingabebilds des liegenden Säuglings mit einem Objekterkennungsmodell, um einen Rahmen für sowohl den Kopf als auch den Oberkörper des Säuglings zu erzeugen;
wobei Bestimmen einer Körperachse basierend auf dem erkannten Kopf und Oberkörper Folgendes umfasst:
Bestimmen einer Körperachse basierend auf den erzeugten Rahmen, sodass die Körperachse durch den Massenschwerpunkt jedes Rahmens verläuft.

3. Verfahren nach Anspruch 1 oder 2, weiter umfassend:
Verarbeiten des Eingabebilds mit einem zweiten Schlüsselpunkterkennungsmodell, um ein oder mehrere Gesichtsmerkmale des Säuglings zu erkennen,
und wobei Bestimmen der Körperhaltung des Säuglings weiter auf dem einen oder den mehreren erkannten Gesichtsmerkmalen basiert,
und wobei optional Bestimmen der Körperhaltung des Säuglings Folgendes umfasst:
Bestimmen, ob das eine oder die mehreren erkannten Gesichtsmerkmale des Säuglings Augen oder Nase des Säuglings umfassen; und
Klassifizieren der Körperhaltung des Säuglings in eine von einer Vielzahl von Körperhaltungsklassifizierungen infolge des Bestimmens, dass das eine oder die mehreren erkannten Gesichtsmerkmale des Säuglings Augen oder Nase des Säuglings umfassen.

4. Verfahren nach einem der Ansprüche 1 bis 3, wobei Bestimmen der Körperachse ein Bestimmen einer Orientierung der Körperachse umfasst, und wobei Bestimmen der Körperhaltung des Säuglings weiter auf der bestimmten Orientierung der Körperachse basiert.

5. Verfahren nach einem der Ansprüche 1 bis 4, weiter umfassend:
Verarbeiten des Eingabebilds mit dem ersten Schlüsselpunkterkennungsmodell, um mindestens eine Hand des Säuglings zu erkennen und zu identifizieren;
und wobei Bestimmen der Körperhaltung des Säuglings weiter auf der bestimmten Körperachse und der Orientierung der/den erkannten und identifizierten Hand/Händen relativ zur Körperachse basiert.

6. Verfahren nach einem der Ansprüche 1 bis 5, wobei Verarbeiten eines Eingabebilds des Säuglings mit dem Objekterkennungsmodell Folgendes umfasst:
Eingeben des Eingabebilds in ein erstes maschinelles Lernmodell, wobei das erste maschinelle Lernmodell trainiert ist, Koordinaten von Rahmen vorherzusagen, die die räumliche Position des Kopfs und Oberkörpers des Säuglings im Eingabebild darstellen,
und wobei vorzugsweise das erste maschinelle Lernmodell unter Verwendung eines Deep-Learning-Algorithmus trainiert wird, der so konfiguriert ist, dass er ein Array von Trainingseingaben und entsprechenden bekannten Ausgaben empfängt, wobei eine Trainingseingabe ein Trainingsbild eines Säuglings umfasst, der in einem Bett liegt oder schläft, und eine entsprechende bekannte Ausgabe die Koordinaten der Rahmen umfasst, die die räumliche Position des Kopfs und des Oberkörpers des Säuglings im Trainingsbild darstellen.

7. Verfahren nach einem der Ansprüche 1 bis 6, wobei Verarbeiten des Eingabebilds mit dem ersten Schlüsselpunkterkennungsmodell Folgendes umfasst:
Eingeben des Eingabebilds in ein zweites maschinelles Lernmodell, wobei das zweite maschinelle Lernmodell trainiert ist, die Koordinaten zu identifizieren und vorherzusagen, die die räumliche Position der Schulter(n) und Hände des Säuglings im Eingabebild darstellen,
und wobei vorzugsweise das zweite maschinelle Lernmodell unter Verwendung eines Deep-Learning-Algorithmus trainiert wird, der so konfiguriert ist, dass er ein Array von Trainingseingaben und entsprechenden bekannten Ausgaben empfängt, und wobei eine Trainingseingabe ein Trainingsbild eines Säuglings umfasst, der in einem Bett liegt oder schläft, und eine entsprechende bekannte Ausgabe die Koordinaten der Schulter- und Handschlüsselpunkte des Säuglings in dem Trainingsbild umfasst.

8. Verfahren nach einem der Ansprüche 1 bis 7, wobei Verarbeiten des Eingabebilds mit dem zweiten Schlüsselpunkterkennungsmodell Folgendes umfasst:
Eingeben des Eingabebilds in ein drittes maschinelles Lernmodell, wobei das dritte maschinelle Lernmodell trainiert ist, die Koordinaten zu identifizieren und vorherzusagen, die die räumliche Position eines oder mehrerer Gesichtsmerkmale des Säuglings im Eingabebild darstellen,
und wobei vorzugsweise das dritte maschinelle Lernmodell unter Verwendung eines Deep-Learning-Algorithmus trainiert wird, der so konfiguriert ist, dass er ein Array von Trainingseingaben und entsprechenden bekannten Ausgaben empfängt, und wobei eine Trainingseingabe ein Trainingsbild eines Säuglings umfasst, der in einem Bett liegt oder schläft, und eine entsprechende bekannte Ausgabe die Koordinaten der Gesichtsmerkmalschlüsselpunkte des Säuglings, wie Augen, Nase und Ohren, in dem Trainingsbild umfasst.

9. Verfahren nach Anspruch 6, 7 oder 8, wobei mindestens eines des ersten, des zweiten und des dritten maschinellen Lernmodells ein vortrainiertes Modell umfasst, das weiter anhand von manuell annotierten Bildern eines in einem Bett liegenden oder schlafenden Säuglings trainiert und feinabgestimmt wurde.

10. Verfahren nach einem der Ansprüche 1 bis 9, wobei Bestimmen der Körperhaltung des Säuglings weiter auf einer Historie einer oder mehrerer vorhergesagter Körperhaltungen des Säuglings für einen Zeitraum vor dem Aufnahmezeitpunkt des Eingabebilds des liegenden Säuglings basiert.

11. Verfahren nach einem der Ansprüche 1 bis 10, weiter umfassend:
Verarbeiten eines oder mehrerer historischer Eingabebilder des liegenden Säuglings mit dem Objekterkennungsmodell, um den Kopf und den Oberkörper des Säuglings in jedem des einen oder der mehreren historischen Eingabebilder zu erkennen;
Bestimmen einer zweiten Körperachse des liegenden Säuglings basierend auf dem erkannten Kopf und Oberkörper in jedem des einen oder der mehreren historischen Eingangsbilder, wobei die Körperachse eine Längsrichtung des Oberkörpers des Säuglings in jedem des einen oder der mehreren historischen Eingangsbilder darstellt;
Verarbeiten jedes des einen oder der mehreren historischen Eingabebilder mit dem ersten Schlüsselpunkterkennungsalgorithmusmodell, um mindestens eine Schulter des Säuglings in dem einen oder den mehreren historischen Eingabebildern zu erkennen;
Bestimmen der Körperhaltung des Säuglings in jedem des einen oder der mehreren historischen Eingabebilder basierend auf der bestimmten zweiten Körperachse und der/den erkannten und identifizierten Schulter(n) in dem einen oder den mehreren historischen Eingabebildern; und
Vorhersagen der Körperhaltung des Säuglings basierend auf Folgendem: der bestimmten Körperhaltung des Säuglings im Eingabebild oder dem Ergebnis einer Mehrheitsabstimmung über die bestimmte Körperhaltung des Säuglings in jedem des einen oder der mehreren historischen Eingabebilder.

12. Computerprogrammprodukt, umfassend Anweisungen, die, wenn das Programm von einem Computer ausgeführt wird, den Computer veranlassen, das Verfahren nach einem vorstehenden Anspruch durchzuführen.

13. Prozessoranordnung, die für Folgendes konfiguriert ist:
Verarbeiten eines Eingabebilds eines liegenden Säuglings mit einem Objekterkennungsmodell, um den Kopf und Oberkörper des Säuglings zu erkennen;
Bestimmen einer Körperachse basierend auf dem erkannten Kopf und Oberkörper, wobei die Körperachse die Längsrichtung des Oberkörpers des Säuglings beschreibt;
Verarbeiten des Eingabebilds mit einem ersten Schlüsselpunkterkennungsmodell, um mindestens eine Schulter des Säuglings zu erkennen und zu identifizieren;
Bestimmen einer Ausrichtung der identifizierten Schulter(n) in Bezug auf die Körperachse; und
Klassifizieren der Körperhaltung des Säuglings in eine von einer Vielzahl von Körperhaltungsklassifizierungen basierend auf der bestimmten Orientierung der identifizierten Schulter(n) relativ zur Körperachse.

14. System (700) zum Bestimmen der Körperhaltung eines liegenden Säuglings, wobei das System Folgendes umfasst:
die Prozessoranordnung nach Anspruch 13, wobei es sich bei der Prozessoranordnung um eine Bildverarbeitungseinheit (720) handelt.

15. Säuglingsüberwachungssystem, umfassend:
das System nach Anspruch 14; und
eine Ausgabeschnittstelle (740), die so konfiguriert ist, dass sie eine Anzeige der bestimmten Körperhaltung des Säuglings ausgibt.

## Revendications

1. Procédé de détermination de la posture corporelle d'un nourrisson couché, le procédé comprenant :
le traitement (610) d'une image d'entrée du nourrisson couché avec un modèle de détection d'objet pour détecter la tête et le haut du torse du nourrisson ;
la détermination (620) d'un axe corporel sur la base de la tête et du haut du torse détectés, dans lequel l'axe corporel décrit la direction longitudinale du haut du torse du nourrisson ;
le traitement (630) de l'image d'entrée avec un premier modèle de détection de points clés pour détecter et identifier au moins une épaule du nourrisson ;
la détermination d'une orientation de la ou des épaules identifiées par rapport à l'axe corporel ; et
le classement de la posture corporelle du nourrisson dans l'une d'une pluralité de classifications de posture corporelle sur la base de l'orientation déterminée de la ou des épaules identifiées par rapport à l'axe corporel.

2. Procédé selon la revendication 1, dans lequel le traitement d'une image d'entrée d'un nourrisson couché à l'aide d'un modèle de détection d'objet pour détecter la tête et le haut du torse du nourrisson comprend :
le traitement de l'image d'entrée du nourrisson couché avec un modèle de détection d'objet pour générer une zone de délimitation pour chacun de la tête et du haut du torse du nourrisson respectivement ; et
dans lequel la détermination d'un axe corporel sur la base de la tête et du haut du torse détectés comprend :
la détermination d'un axe corporel à partir des zones de délimitation générées, de telle sorte que l'axe corporel passe par le centre de masse de chaque zone de délimitation.

3. Procédé selon la revendication 1 ou 2 comprenant en outre :
le traitement de l'image d'entrée avec un deuxième modèle de détection de points clés pour détecter une ou plusieurs caractéristiques faciales du nourrisson,
et dans lequel la détermination de la posture corporelle du nourrisson est en outre basée sur les une ou plusieurs caractéristiques faciales détectées,
et, facultativement, dans lequel la détermination de la posture corporelle du nourrisson comprend :
la détermination pour savoir si les une ou plusieurs caractéristiques faciales détectées du nourrisson comprennent les yeux ou le nez du nourrisson ; et
le classement de la posture corporelle du nourrisson dans l'une d'une pluralité de classifications de posture corporelle à la suite de la détermination que les une ou plusieurs caractéristiques faciales détectées du nourrisson comprennent les yeux ou le nez du nourrisson.

4. Procédés selon l'une quelconque des revendications 1 à 3, dans lesquels la détermination de l'axe corporel comprend la détermination d'une orientation de l'axe corporel et dans lesquels la détermination de la posture corporelle du nourrisson est en outre basée sur l'orientation déterminée de l'axe corporel.

5. Procédé selon l'une quelconque des revendications 1 à 4, comprenant en outre :
le traitement de l'image d'entrée avec le premier modèle de détection de points clés pour détecter et identifier au moins une main du nourrisson,
et dans lequel la détermination de la posture corporelle du nourrisson est en outre basée sur l'axe corporel déterminé et l'orientation de la ou des mains détectées et identifiées par rapport à l'axe corporel.

6. Procédé selon l'une quelconque des revendications 1 à 5, dans lequel le traitement d'une image d'entrée du nourrisson avec le modèle de détection d'objet comprend :
la fourniture de l'image d'entrée à un premier modèle d'apprentissage automatique, dans lequel le premier modèle d'apprentissage automatique est entraîné à prédire des coordonnées de zones de délimitation représentant l'emplacement spatial de la tête et du haut du torse du nourrisson dans l'image d'entrée,
et, de préférence, dans lequel le premier modèle d'apprentissage automatique est entraîné à l'aide d'un algorithme d'apprentissage profond configuré pour recevoir un ensemble d'entrées d'entraînement et de sorties connues respectives, dans lequel une entrée d'entraînement comprend une image d'entraînement d'un nourrisson couché ou dormant dans un lit et une sortie connue respective comprend les coordonnées des zones de délimitation représentant l'emplacement spatial de la tête et du haut du torse du nourrisson dans l'image d'entraînement.

7. Procédé selon l'une quelconque des revendications 1 à 6, dans lequel le traitement de l'image d'entrée avec le premier modèle de détection de points clés comprend :
la fourniture de l'image d'entrée à un deuxième modèle d'apprentissage automatique, dans lequel le deuxième modèle d'apprentissage automatique est entraîné à identifier et à prédire les coordonnées représentant l'emplacement spatial de la ou des épaules et des mains du nourrisson dans l'image d'entrée,
et, de préférence, dans lequel le deuxième modèle d'apprentissage automatique est entraîné à l'aide d'un algorithme d'apprentissage profond configuré pour recevoir un ensemble d'entrées d'entraînement et de sorties connues respectives et dans lequel une entrée d'entraînement comprend une image d'entraînement d'un nourrisson couché ou dormant dans un lit et une sortie connue respective comprend les coordonnées des points clés de l'épaule et de la main du nourrisson dans l'image d'entraînement.

8. Procédé selon l'une quelconque des revendications 1 à 7, dans lequel le traitement de l'image d'entrée avec le deuxième modèle de détection de points clés comprend :
la fourniture de l'image d'entrée à un troisième modèle d'apprentissage automatique, dans lequel le troisième modèle d'apprentissage automatique est entraîné à identifier et à prédire les coordonnées représentant l'emplacement spatial d'une ou de plusieurs caractéristiques faciales du nourrisson dans l'image d'entrée,
et, de préférence, dans lequel le troisième modèle d'apprentissage automatique est entraîné à l'aide d'un algorithme d'apprentissage profond configuré pour recevoir un ensemble d'entrées d'entraînement et de sorties connues respectives et dans lequel une entrée d'entraînement comprend une image d'entraînement d'un nourrisson couché ou dormant dans un lit et une sortie connue respective comprend les coordonnées des points clés des caractéristiques faciales du nourrisson tels que les yeux, le nez et les oreilles dans l'image d'entraînement.

9. Procédé selon la revendication 6, 7 ou 8, dans lequel au moins un des premier, deuxième et troisième modèles d'apprentissage automatique comprend un modèle pré-entraîné, en outre entraîné et affiné sur des images annotées manuellement d'un nourrisson couché ou dormant dans un lit.

10. Procédé selon l'une quelconque des revendications 1 à 9, dans lequel la détermination de la posture corporelle du nourrisson est en outre basée sur un historique d'une ou de plusieurs postures corporelles prédites du nourrisson pendant une durée précédant le moment de la capture de l'image d'entrée du nourrisson couché.

11. Procédé selon l'une quelconque des revendications 1 à 10, comprenant en outre :
le traitement d'une ou de plusieurs images d'entrée historiques du nourrisson couché avec le modèle de détection d'objet pour détecter la tête et le haut du torse du nourrisson dans chacune des une ou plusieurs images d'entrée historiques ;
la détermination d'un second axe corporel du nourrisson couché sur la base de la tête et du haut du torse détectés dans chacune des une ou plusieurs images d'entrée historiques, l'axe corporel représentant une direction longitudinale du haut du torse du nourrisson dans chacune des une ou plusieurs images d'entrée historiques ;
le traitement de chacune des une ou plusieurs images d'entrée historiques avec le premier modèle d'algorithme de détection de points clés pour détecter et identifier au moins une épaule du nourrisson dans les une ou plusieurs images d'entrée historiques ;
la détermination de la posture corporelle du nourrisson dans chacune des une ou plusieurs images d'entrée historiques, sur la base du second axe corporel déterminé et de la ou des épaules détectées et identifiées dans les une ou plusieurs images d'entrée historiques ; et
la prédiction de la posture corporelle du nourrisson sur la base : de la posture corporelle déterminée du nourrisson dans l'image d'entrée ou du résultat d'un vote majoritaire sur la posture corporelle déterminée du nourrisson dans chacune des une ou plusieurs images d'entrée historiques.

12. Programme informatique comprenant des instructions qui, lorsque le programme est exécuté par un ordinateur, amènent l'ordinateur à réaliser le procédé selon une quelconque revendication précédente.

13. Agencement de processeur configuré pour :
traiter une image d'entrée d'un nourrisson couché avec un modèle de détection d'objet pour détecter la tête et le haut du torse du nourrisson ;
déterminer un axe corporel sur la base de la tête et du haut du torse détectés, dans lequel l'axe corporel décrit la direction longitudinale du haut du torse du nourrisson ;
traiter l'image d'entrée avec un premier modèle de détection de points clés pour détecter et identifier au moins une épaule du nourrisson ;
déterminer une orientation de la ou des épaules identifiées par rapport à l'axe corporel ; et
classer la posture corporelle du nourrisson dans l'une d'une pluralité de classifications de posture corporelle sur la base de l'orientation déterminée de la ou des épaules identifiées par rapport à l'axe corporel.

14. Système (700) pour déterminer la posture corporelle d'un nourrisson couché, le système comprenant :
l'agencement de processeur selon la revendication 13, dans lequel l'agencement de processeur est une unité de traitement d'image (720).

15. Système de surveillance de nourrisson comprenant :
le système selon la revendication 14 ; et
une interface de sortie (740) configurée pour délivrer en sortie une indication de la posture corporelle déterminée du nourrisson.
